Europäisches Patentamt

European Patent Office

Office européen des brevets

(11) Veröffentlichungsnummer: **0 365 704**
**A1**

## EUROPÄISCHE PATENTANMELDUNG

(21) Anmeldenummer: 88117868.5

(22) Anmeldetag: 26.10.88

(51) Int. Cl.⁵: **A61K 37/12**

(43) Veröffentlichungstag der Anmeldung:
**02.05.90 Patentblatt 90/18**

(84) Benannte Vertragsstaaten:
**AT CH DE FR GB GR LI**

(71) Anmelder: **ZENTRALNA PROBLEMNA
LABORATORIA PO KRYOBIOLOGIA I
LYOPHILISAZIA
Boul. Tcherni Vrah Nr. 65
BG-1407 Sofia(BG)**

(72) Erfinder: **Tzvetkov, Tzvetan Dimitrov
54-B Iliya Filipov Strasse
Sofia(BG)**
Erfinder: **Atanassov, Atanas Tanev, Dr.
Zdanov Strasse 120-B
Sofia(BG)**
Erfinder: **Velitchkov, Nado Georgiev, Dr.**

**Kompl. V. Ivanov, Bl. 310-2
Sofia(BG)**
Erfinder: **Metchkarski, Stefan Ninov, Dr.
Viktor Grigorovitch Strasse 12
Sofia(BG)**
Erfinder: **Tarlov, Pavel Kamenov
Ami Bue Strasse Nr. 82
Sofia(BG)**
Erfinder: **Kavardijkova, Velitchka Atanassova,
Dr.
Djovani Gorini Strasse Nr. 4
Sofia(BG)**

(74) Vertreter: **von Füner, Alexander, Dr. et al
Patentanwälte v. Füner, Ebbinghaus, Finck
Mariahilfplatz 2 & 3
D-8000 München 90(DE)**

(54) **Biopräparat für Hämostase bei Magenblutungen.**

(57) Das Biopräparat zur Hämostase bei Magenblutungen enthält Kollagenlösung, Aluminiumhydroxid-Gel, Magnesiumhydroxid-Gel, D-Sorbitol und Epsylon-Aminocapronsäure in folgendem Verhältnis in Gewichts-%:

| | | | |
|---|---|---|---|
| Kollagenlösung | 24,0 | bis | 63,0% |
| Aluminiumhydroxid-Gel | 32,0 | bis | 70,0% |
| Magnesiumhydroxid-Gel | 1,0 | bis | 3,0% |
| D-Sorbitol | 0,5 | bis | 2,0% |
| Epsylon-Aminocapronsäure | 1,0 | bis | 3,0%. |

Das Biopräparat zeigt eine schnelle und dauerhafte hämostatische Wirkung bei Magenblutungen und findet in der Humanmedizin breite Anwendung.

EP 0 365 704 A1

## BIOPRÄPARAT ZUR HÄMOSTASE BEI MAGENBLUTUNGEN

Die Erfindung betrifft ein Biopräparat zur Hämostase bei Magenblutungen, das in der Humanmedizin verwendet wird.

Es ist ein lyophilisiertes Präparat bekannt, das Thrombin, Natriumdehydrogenphosphat, Magnesiumhydroxid und entfettete Milch in Pulverform zur Hämostase bei Magenblutungen enthält (Handbuch für Arzneimittel, 1982, Seite 823).

Es ist auch die Verwendung von Kollagenlösungen bei Magenblutungen bekannt (V.V. Kovanov, I.A. Sitschenikov, "Kollagenplastik in der Medizin", 1978).

Der Nachteil dieser Präparate liegt darin, daß sie keine hämostatische Wirkung von ausreichender Zuverlässigkeit besitzen.

Der Erfindung liegt die Aufgabe zugrunde, ein Biopräparat zur Hämostase bei Magenblutungen zu entwickeln, das eine schnelle und dauerhafte hämostatische Wirkung durch Bildung eines Filmüberzugs auf der Magenschleimhaut hervorruft.

Diese Aufgabe wird durch die Entwicklung eines Biopräparates zur Hämostase bei Magenblutungen mit folgenden Bestandteilen (in Gewichts-%) gelöst:

| Kollagenlösung | 24,0% | bis | 63,0% |
|---|---|---|---|
| Aluminiumhydroxid-Gel | 32,0% | bis | 70,0% |
| Magnesiumhydroxid-Gel | 1,0% | bis | 3,0% |
| D-Sorbitol | 0,5% | bis | 2,0% |
| Epsilon-Aminocapronsäure | 1,0% | bis | 3,0%. |

Das Präparat wird durch Mischen der einzelnen Bestandteile bei Zimmertemperatur bereitet. Die dazu verwendete Kollagenlösung wurde durch Enzymhydrolyse mit Sauerprotease aufbereitet.

Der Vorteil des Biopräparates besteht darin, daß es eine schnelle und beständige Hämostase bei Magenblutungen aufweist. Das Präparat bildet einen Schutzüberzug auf der Magenschleimhaut und erlaubt eine fortdauernde Wirkung der einzelnen Bestandteile, neutralisiert die Salzsäure im Magen, besitzt eine dauerhafte Antiazidwirkung, wird nicht durch den Organismus resorbiert und besitzt weiterhin einen schmerzlindernden Effekt.

Beispiel 1

| Kollagenlösung (4%ig) | 48,0% |
|---|---|
| Aluminiumhydroxid-Gel | 47,0% |
| Magnesiumhydroxid-Gel | 2,0% |
| D-Sorbitol | 1,0% |
| Epsilon-Aminocapronsäure | 2,0% |

Das Biopräparat wird bereitet, indem man zur Lösung des mikrokristallinen Kollagens aufeinanderfolgend bei ununterbrochener Homogenisierung Aluminiumhydroxid-Gel, Magnesi umhydroxid-Gel, D-Sorbitol und Epsilon-Aminocapronsäure hinzufügt. Die so erhaltene homogene Mischung wird in Glasflacons von 100 ml dosiert.

Beispiel 2

| Kollagenlösung (4%ig) | 24,0% |
|---|---|
| Aluminiumhydroxid-Gel | 70,0% |
| Magnesiumhydroxid-Gel | 3,0% |
| D-Sorbitol | 2,0% |

Das Biopräparat wird nach Beispiel 1 bereitet.

Beispiel 3

| Kollagenlösung (4%ig) | 63,0% |
|---|---|
| Aluminiumhydroxid-Gel | 32,0% |
| Magnesiumhydroxid-Gel | 1,0% |
| D-Sorbitol | 0,5% |
| Epsilon-Aminocapronsäure | 3,0% |

Das Biopräparat wird nach Beispiel 1 bereitet.

Beispiel 4

Das Biopräparat zur Hämostase bei Magenblutungen wurde in der Klinik an mehr als 30 Kranken geprüft. Die klinischen Angaben zeigen, daß das Präparat einen sicheren und dauerhaften Effekt als hämostatisches Mittel bei Magenblutungen bei Kranken mit Ulcus duodeni - Hämorrhagicum, Ulcus ventriculi-Hämorrhagicum, Gastritis-Hämorrhagicum, Syndrom Mallori-Weiss und Neoventriculi-Hämorrhagicum aufweist. Bei allen Kranken zeigt das Präparat einen starken und dauerhaften hämostatischen Effekt. Bei der Verwendung des Biopräparates wurden keine Nebenwirkungen beobachtet.

**Ansprüche**

Biopräparat zur Hämostase bei Magenblutungen, das eine Kollagenlösung enthält, **gekennzeichnet dadurch,** daß es zusätzlich noch Aluminiumhydroxid-Gel, Magnesiumhydroxid-Gel, D-Sorbitol und Epsilon-Aminocapronsäure in folgendem Verhältnis (in Gewichts-%) enthält:

| Kollagenlösung | 24,0 | bis | 63,0% |
|---|---|---|---|
| Aluminiumhydroxid-Gel | 32,0 | bis | 70,0% |
| Magnesiumhydroxid-Gel | 1,0 | bis | 3,0% |
| D-Sorbitol | 0,5 | bis | 2,0% |
| Epsylon-Aminocapronsäure | 1,0 | bis · | 3,0%. |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-4 006 220 (S.K. GOTTLIEB) <br> * Ansprüche 1,3,17 * <br> --- | | A 61 K 37/12 // <br> (A 61 K 37/12 <br> A 61 K 33:08 <br> A 61 K 31:045 <br> A 61 K 31:195) |
| A | EP-A-0 109 188 (SERONO PHARMACEUTICAL PARTNERS) <br> * Ansprüche 1,6 * <br> ----- | | |

RECHERCHIERTE
SACHGEBIETE (Int. Cl.5)

A 61 K

Der vorliegende Recherchenbericht wurde für alle Patentansprüche erstellt

| Recherchenort | Abschlußdatum der Recherche | Prüfer |
|---|---|---|
| DEN HAAG | 29-06-1989 | PEETERS J.C. |